# EUROPEAN PATENT APPLICATION

(11) **EP 4 471 136 A1**
(43) Date of publication of application: **04.12.2024**
(21) Application number: 23746498.7
(22) Date of filing: 30.01.2023
(51) Int. Cl.: C12N 9/06, C12N 15/53, C12N 15/70, C12P 9/00, C12P 13/04

(54) **D-AMINO ACID OXIDASE AND USE THEREOF IN PREPARATION OF L-PHOSPHINOTHRICIN OR INTERMEDIATE THEREOF**

(30) Priority: 30.01.2022 CN 202210114692
(71) Applicant: Shanghai Qizhou Ziyue Biotechnology Co., LTD, Shanghai 201100 (CN)
(72) Inventor: JIAO, Qi, Shanghai 200241 (CN); WANG, Shu, Shanghai 200241 (CN); TIAN, Zhenhua, Shanghai 200241 (CN); CHENG, Zhanbing, Shanghai 200241 (CN); MA, Shuai, Shanghai 200241 (CN); CHENG, Jing, Shanghai 200241 (CN)
(74) Representative: dompatent von Kreisler Selting Werner - Partnerschaft von Patent- und Rechtsanwälten mbB
(86) International application number: PCT/CN2023/073897
(87) International publication number: WO 2023/143621

(57) **Abstract**

Provided are a D-amino acid oxidase and use thereof in the preparation of L-phosphinothricin or an intermediate thereof. Provided is a D-amino acid oxidase having an amino acid sequence comprising an amino acid residue difference as compared to SEQ ID NO: 1, the amino acid residue difference being selected from one or a plurality of: K29G/H/I/N/Q/W/Y/C/L; V42C/D/E/H/Y; E195N/Y/Q; C234L; and V326W. The activity and/or thermal stability of the D-amino acid oxidase is not lower than that of a D-amino acid oxidase having an amino acid sequence as set forth in SEQ ID NO: 1. Provided is a D-amino acid oxidase with higher thermal stability. The operating temperature range of the enzyme is expanded while the activity of the enzyme is improved. The enzyme can have a prolonged service life when used at a relatively low temperature, and can have an improved catalytic efficiency when used at a relatively high temperature.

## Description

The present application claims the right of priority of Chinese Patent Application 2022101146927 filed on January 30, 2022. This Chinese patent application is incorporated herein by reference in its entirety.

### TECHNICAL FIELD

The present disclosure belongs to the field of biotechnology, and specifically relates to a D-amino acid oxidase and use thereof in the preparation of L-glufosinate or an intermediate thereof.

### BACKGROUND

Glufosinate, glyphosate and paraquat are known as three major herbicides in the world, and they are all non-selective. Among them, due to the extremely strong toxicity of paraquat, its use has been strictly restricted or banned in many countries in recent years; glyphosate is widely used, but it has been removed from the approved list of Grade A green foods in China. Glufosinate has the characteristics of high activity, broad herbicidal spectrum, and good environmental compatibility, exerting herbicidal activity faster than that of glyphosate. It can also be used in areas where weeds are resistant to glyphosate, therefore glufosinate has great market development potential and broad application prospects.

In 2020, the global market size of glufosinate reached US$1.05 billion. The global market compound growth rate in the past 6 years was 6.3%, making it the fastest-growing non-selective herbicide. Therefore, developing or optimizing the L-glufosinate production process is of great significance to improve economic benefits, reduce usage costs, and reduce environmental pressure.

Commercially used glufosinate is a mixture of racemates, with two isomers, D and L. However, only the L form is active, and is easily decomposed in soil with low toxicity. There are two main methods for producing L- glufosinate: a chemical method and a biological method. The chemical production of glufosinate mainly involves the Strecker process and the thermal cracking-ACA process. The Srecker process is complex, involves flammable and explosive raw materials, and low yield. The thermal cracking-ACA process is complex, and methyl dichlorophosphate (MDP) is the key intermediate at the front end of this process and the technical barrier to the process. In terms of the biological method, the reaction conditions are mild, the production process is simple, and the products are easily separated. However, the yield is relatively low and the cost is relatively high, and it has not been industrialized yet.

In CN 105603015A, 2-oxo-4-(hydroxymethylphosphinyl)butyric acid (PPO) and salts thereof are used as substrates, alanine is used as an amino donor, and an *ex vivo* transaminase or a cell expressing a transaminase *in vitro* is used to catalyze the substrate to undergo a transamination reaction with alanine to obtain L-glufosinate. In CN 106978453B, a method for preparing L-glufosinate using amino acid dehydrogenase was provided. The method uses 2-carbonyl-4-(hydroxymethylphosphinyl)butyric acid or a salt thereof as a substrate to perform a transamination reaction to obtain L-glufosinate. This method has a high raw material conversion rate, but the substrate synthesis process is complex and the cost is high.

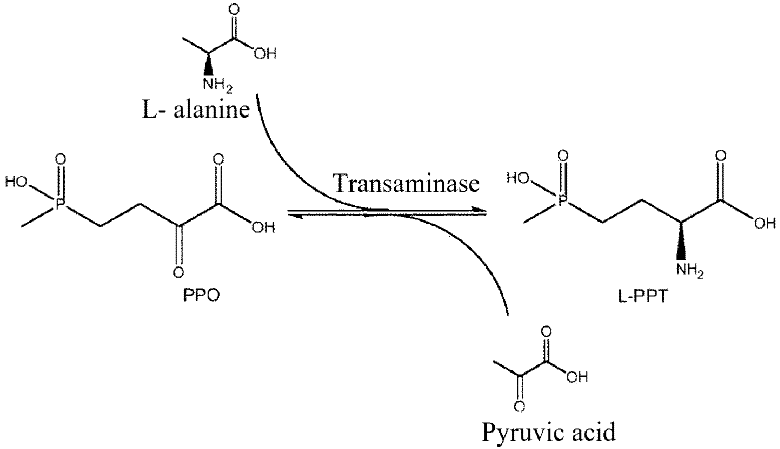

In US 9834802B2, D,L-glufosinate is used as raw material to generate an intermediate product PPO with the catalysis of a D-amino acid oxidase mutant, and L-glufosinate is generated from PPO with the catalysis of an amino acid transaminase. The composition obtained from the reaction according to this method contains L-glufosinate, D-glufosinate, and PPO, in which the L-glufosinate content reaches 80% or more, the D-glufosinate content is less than 10%, and the PPO content is less than 20%. However, the stability of enzyme activity in the method still needs to be improved.

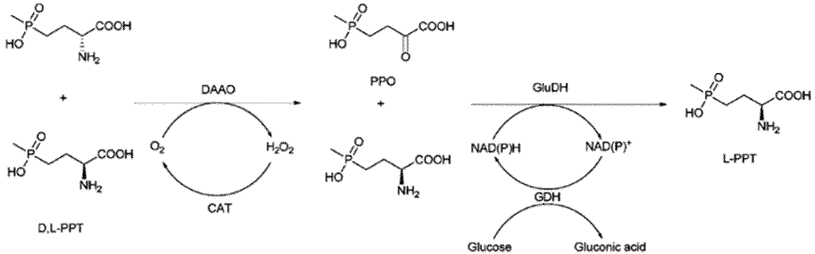

Therefore, there is an urgent need to find a D-amino acid oxidase with high stability of enzyme activity, high D-glufosinate conversion rate, and high ee value of L-glufosinate.

### CONTENT OF THE PRESENT INVENTION

The technical problem to be solved by the present disclosure is to overcome the shortcomings of D-amino acid oxidase in the prior art such as low enzyme activity, poor stability of enzyme activity and low L-glufosinate yield. In order to solve the above technical problems, the present disclosure provides a D-amino acid oxidase and use thereof in the preparation of L-glufosinate or an intermediate thereof. When using a D-amino acid oxidase with higher thermal stability, the operating temperature range of the enzyme is expanded while the activity of the enzyme is improved, and when used at a relatively low temperature, the enzyme can have a prolonged service life; when used at a relatively high temperature, the enzyme can have an improved catalytic efficiency. Enzymes with high thermal stability have the advantages of increased chemical reaction rates, improved product quality, stable activity, and long storage time.

A first aspect of the present disclosure provides a D-amino acid oxidase, wherein the amino acid sequence of the D-amino acid oxidase has one or more amino acid residue difference selected from the following as compared to SEQ ID NO: 1:

K29G/H/I/N/Q/W/Y/C/L; V42C/D/E/H/Y; E195N/Y/Q; C234L; V326W;
and the activity and/or thermal stability of the D-amino acid oxidase is not lower than that of a D-amino acid oxidase of the amino acid sequence as set forth in SEQ ID NO: 1.

In the present disclosure, the "/" between amino acid residues represents different selections of amino acid residues at the positions corresponding to the amino acid residues. For example, E195N/Y/Q means that the difference at position 195 can be N, Y or Q.

In some preferred embodiments of the present disclosure, the amino acid sequence of the D-amino acid oxidase includes two or more amino acid residue differences selected from the following as compared to SEQ ID NO: 1: V42Y, E195Y, V326W, C234L.

Preferably, the D-amino acid oxidase of the present disclosure includes the amino acid residue difference of C234L as compared to SEQ ID NO: 1, and further includes one or two amino acid residue differences of V42Y, E195Y and V326W.

More preferably, the D-amino acid oxidase comprises the amino acid residue difference selected from any of the following groups as compared to SEQ ID NO: 1:
C234L and V42Y;
C234L and E195Y;
C234L and V326W;
C234L, K29C and V42Y;
C234L, K29G and V42Y;
C234L, K29L and V42Y;
C234L, V326W and V42Y;
C234L, V42Y and E195Y;
and C234L, E195Y and V326W.

In other preferred embodiments of the present disclosure, the D-amino acid oxidase includes the amino acid difference of E195Y as compared to SEQ ID NO: 1, and further includes the amino acid residue difference of V42Y or V326W;
preferably, the D-amino acid oxidase comprises the amino acid residue difference selected from any of the following groups as compared to SEQ ID NO: 1: E195Y and V42Y;
E195Y and V326W;
E195Y, K29Q and V42Y;
E195Y, K29W and V42Y;
and E195Y, K29Y and V42Y

In other preferred embodiments of the present disclosure, the D-amino acid oxidase comprises an amino acid residue difference selected from any of the following groups as compared to SEQ ID NO: 1:
K29G; K29H; K29I; K29N; K29Q; K29W; K29Y; V42C; V42D; V42E; V42H; V42P; V42Y; E195H; E195N; E195Y; E195Q; C234L; and V326W.

A second aspect of the present disclosure provides an isolated nucleic acid encoding the D-amino acid oxidase according to the first aspect of the present disclosure.

A third aspect of the present disclosure provides a recombinant expression vector comprising the nucleic acid according to the second aspect of the present disclosure.

A fourth aspect of the present disclosure provides a transformant comprising the nucleic acid according to the second aspect of the present disclosure or the recombinant expression vector according to the third aspect of the present disclosure.

The host cell of the transformant of the present disclosure can be conventional in the art, preferably *Escherichia coli*, such as *E.coli* BL21 (DE3).

A fifth aspect of the present disclosure provides a method for preparing the D-amino acid oxidase according to the present disclosure, comprising culturing the transformant according to the fourth aspect of the present disclosure under a condition suitable for expressing the D-amino acid oxidase.

A sixth aspect of the present disclosure provides a method for preparing 2-oxo-4-(hydroxymethylphosphinyl)butyric acid or a salt thereof, including the following steps: in the presence of the D-amino acid oxidase according to the first aspect of the present disclosure, subjecting the substrate to an oxidation reaction to obtain the 2-oxo-4-(hydroxymethylphosphinyl)butyric acid or the salt thereof.

The substrate is preferably D-glufosinate or a salt thereof. The D-glufosinate or the salt thereof can exist alone or together with L-glufosinate or a salt thereof. For example, the substrate can exist as racemic glufosinate or a salt thereof.

The oxidation reaction is preferably carried out under ventilation conditions; the ventilation is preferably performed with air or oxygen; and the ventilation rate is preferably 0.5VVM-1.5VVM such as 1VVM.

The oxidation reaction is preferably carried out in the presence of a catalase.

The D-amino acid oxidase preferably exists in the form of bacterial cells containing D-amino acid oxidase, crude enzyme, pure enzyme or immobilized enzyme of D-amino acid oxidase.

The concentration of the substrate is preferably 0.1-0.5 mol/L; preferably, 0.17 mol/L.

The pH of the reaction system of the oxidation reaction is preferably 7-9, such as 8.

The temperature of the reaction system of the oxidation reaction is preferably 20-50°C, such as 25°C.

In some preferred embodiments, the mass ratio of the bacterial cells containing the D-amino acid oxidase to the substrate is 1 : (0.5-3), for example, 1:1.

In some more preferred embodiments, the mass ratio of the catalase to the bacterial cells containing the D-amino acid oxidase is 1 : (20-60); for example, 1 : 40.

The reaction system of the oxidation reaction preferably further includes a buffer. The buffer can be conventional in the art, and is preferably a phosphate buffer, such as ammonium dihydrogen phosphate and diammonium hydrogen phosphate, disodium hydrogen phosphate and sodium dihydrogen phosphate.

The buffer is used to regulate the pH of the reaction system. Preferably, the pH of the buffer is 8.0.

A seventh aspect of the present disclosure provides a method for preparing L-glufosinate or a salt thereof, including the following steps:
(1) obtaining 2-oxo-4-(hydroxymethylphosphinyl)butyric acid or a salt thereof by adopting the preparation method according to the sixth aspect of the present disclosure;
(2) in the presence of glutamate dehydrogenase, inorganic amino donor and reduced coenzyme, performing an ammoniation reaction with the 2-oxo-4-(hydroxymethylphosphinyl)butyric acid or the salt thereof obtained in step (1) to obtain the L-glufosinate or the salt thereof.

In the method for preparing L-glufosinate or the salt thereof according to the present disclosure, the reduced coenzyme is preferably NADPH or NADH.

In the method for preparing L-glufosinate or the salt thereof according to the present disclosure, the inorganic amino donor is preferably one or more of ammonia, ammonium sulfate, ammonium chloride, diammonium hydrogen phosphate, ammonium acetate, ammonium formate and ammonium bicarbonate, and the form of the ammonia for use is preferably ammonia water.

In the method for preparing L-glufosinate or the salt thereof according to the present disclosure, the pH of the reaction system of the ammoniation reaction is preferably 7-10, and more preferably 8.4-8.6.

In the method for preparing L-glufosinate or the salt thereof according to the present disclosure, the reaction temperature of the ammoniation reaction is preferably 28-35°C, more preferably 30-33°C.

In the method for preparing L-glufosinate or the salt thereof according to the present disclosure, the glutamate dehydrogenase is preferably Mutants 1-4 in CN 201910434350.1.

In the method for preparing L-glufosinate or the salt thereof according to the present disclosure, the mass ratio of the glutamate dehydrogenase to the substrate D-glufosinate is preferably 1 : (0.5-3) , for example 1 : 1.25.

In the method for preparing L-glufosinate or the salt thereof according to the present disclosure, the molar ratio of the substrate D-glufosinate to the inorganic amino donor is preferably 1 : (1-1.5), such as 1 : 1.

The method for preparing L-glufosinate or the salt thereof according to the present disclosure preferably further includes the following step: subjecting the oxidized coenzyme to a reduction reaction to obtain the reduced coenzyme in the presence of a dehydrogenase and a hydrogen donor.

The dehydrogenase is preferably glucose dehydrogenase, alcohol dehydrogenase or formate dehydrogenase.

The hydrogen donor is preferably glucose, isopropyl alcohol or formate.

In some preferred embodiments of the present disclosure, when the dehydrogenase is alcohol dehydrogenase, the hydrogen donor is isopropyl alcohol; when the dehydrogenase is glucose dehydrogenase, the hydrogen donor is glucose; when the dehydrogenase is formate dehydrogenase, the hydrogen donor is formate.

The method for preparing L-glufosinate or the salt thereof according to the present disclosure can be carried out in steps, for example, first performing an oxidation reaction to prepare 2-oxo-4-(hydroxymethylphosphinyl)butyric acid or a salt thereof, and then performing ammoniation reaction to prepare L-glufosinate or the salt thereof; the method can also be carried out by a "one-pot method", for example, mixing all raw materials to prepare L-glufosinate or the salt thereof.

An eighth aspect of the present disclosure provides a use of the D-amino acid oxidase according to the first aspect of the present disclosure in the preparation of L-glufosinate or a salt thereof, and 2-oxo-4-(hydroxymethylphosphinyl)butyric acid or a salt thereof.

The DAAO enzyme and D-amino acid oxidase mentioned in the present disclosure can be used interchangeably.

In the present disclosure, the amount of substrate for D-amino acid oxidase is calculated with D-glufosinate or a salt thereof.

On the basis of conforming to common knowledge in the art, the above-mentioned preferred conditions can be arbitrarily combined to obtain various preferred embodiments of the present disclosure.

Reagents and raw materials used in the present disclosure are all commercially available.

The positive effects of the present disclosure are: provided is a D-amino acid oxidase with higher thermal stability, the operating temperature range of which is expanded while the activity of the enzyme is improved. The enzyme can have a prolonged service life when used at a relatively low temperature, and can have an improved catalytic efficiency when used at a relatively high temperature.

### DETAILED DESCRIPTION OF THE PREFERRED EMBODIMENT

The present disclosure is further described below by way of examples; however, the present disclosure is not limited to the scope of the described examples. For the experimental methods in which no specific conditions are specified in the following examples, selections are made according to conventional methods and conditions or according to the product instructions.

The chiral analysis and concentration analysis of the product L-glufosinate were carried out by pre-column derivatization high-performance liquid chromatography. The specific analysis method is:
(1) Chromatographic conditions: Agilent ZORBAX Eclipse plus C18, 3.5 µm, 150*4.6 mm. Mobile phase A: 0.1% TFA+H₂O, mobile phase B: 0.1%TFA+CAN. Detection wavelength: 340 nm, flow rate: 1.0 mL/min, column temperature: 30°C.
(2) Derivatization reagents: Marfey's reagent
(3) Derivative reactions: 50 mg of the test sample was weighed and added into a 25 ml volumetric flask, 15 ml of diluent (pure water:acetonitrile = 50 : 50) was added and sonicated for 5 minutes, purified water was then added for dilution to the mark, and mixed well. 1 mL of the above solution was weighed into a 5 mL volumetric flask, 1 mL of Marfey's reagent solution and 0.1 mL of sodium bicarbonate (1 M) solution were added, the volumetric flask was covered with a lid and the mixture was heated in a 50°C oven in the dark for 1 h. After the reaction was completed, 0.1 mL of hydrochloric acid solution was added and mixed well.

1 mL of the above mixed solution was taken, 4 mL of the diluent was added, mixed well, and poured into an injection bottle. 10 µL was injected for analysis.

PPO was analyzed by ion pair chromatography. The specific analysis method was:

Chromatographic conditions: MLtimate AQ-C18, 5 µm, 4.6*250 mm; mobile phase: 0.05 mol/L diammonium hydrogen phosphate PH = 3.6 : 10% tetrabutylammonium hydroxide aqueous solution: acetonitrile = 91 : 1 : 8; Detection wavelength: 205 nm; flow rate: 1.0 mL/min; column temperature: 25°C.
sample: 5 mg/mL H₂O solution. 10 µL was injected for analysis.

The experimental methods in the present disclosure are all conventional methods unless otherwise specified. For specific gene cloning operations, please refer to the "Molecular Cloning: A Laboratory Manual" edited by J. Sambrook *et al.*

The abbreviated symbols of amino acids in the present disclosure are conventional in the art unless otherwise specified. The specific amino acids corresponding to the abbreviated symbols are shown in Table 1.

**Table 1**

| Amino acid name | Three letter symbol | Single letter symbol | Amino acid name | Three letter symbol | Single letter symbol |
|---|---|---|---|---|---|
| Alanine | Ala | A | Leucine | Leu | L |
| Arginine | Arg | R | Lysine | Lys | K |
| Asparagine | Asn | N | Methionine | Met | M |
| Aspartic acid | Asp | D | Phenylalanine | Phe | F |
| Cysteine | Cys | C | Proline | Pro | P |
| Glutamine | Gln | Q | Serine | Ser | S |
| Glutamic acid | Glu | E | Threonine | Thr | T |
| Glycine | Gly | G | Tryptophan | Trp | W |
| Histidine | His | H | Tyrosine | Tyr | Y |
| Isoleucine | Ile | I | Valine | Val | V |

The codons corresponding to the amino acids are also routine in the art. The specific correspondence between amino acids and codons is shown in Table 2.

**Table 2**

| First nucleotide | Second nucleotide | | | | Third nucleotide |
|---|---|---|---|---|---|
| | T | C | A | G | |
| T | Phenylalanine F | Serine S | Tyrosine Y | Cysteine C | T |
| | Phenylalanine F | Serine S | Tyrosine Y | Cysteine C | C |
| | Leucine L | Serine S | Stop codon | Stop codon | A |
| | Leucine L | Serine S | Stop codon | Tryptophan W | G |
| C | Leucine L | Proline P | Histidine H | Arginine R | T |
| | Leucine L | Proline P | Histidine H | Arginine R | C |
| | Leucine L | Proline P | Glutamine Q | Arginine R | A |
| | Leucine L | Proline P | Glutamine Q | Arginine R | G |
| A | Isoleucine I | Threonine T | Asparagine N | Serine S | T |
| | Isoleucine I | Threonine T | Asparagine N | Serine S | C |
| | Isoleucine I | Threonine T | Lysine K | Arginine R | A |
| | Methionine M | Threonine T | Lysine K | Arginine R | G |
| G | Valine V | Alanine A | Aspartic acid D | Glycine G | T |
| | Valine V | Alanine A | Aspartic acid D | Glycine G | C |
| | Valine V | Alanine A | Glutamic acid E | Glycine G | A |
| | Valine V | Alanine A | Glutamic acid E | Glycine G | G |

pET28a plasmid and bugbuster protein extraction reagent were purchased from Novagen; NdeI enzyme and HindIII enzyme were purchased from Thermo Fisher Company; *E.coli* BL21 competent cells were purchased from Beijing Ding Guo Prosperous Biotechnology Co., Ltd.; catalase was purchased from Shandong Fengtai Biotechnology Co., Ltd.

### Example 1: Preparation of D-amino acid oxidase (DAAO)

The engineered strain for DAAO enzyme was derived from the engineered strain containing seq.79 disclosed in patent CN 111019916B. The amino acid sequence is shown in SEQ ID NO: 1, and the nucleotide sequence is shown in SEQ ID NO: 12.

LB liquid culture medium composition: peptone 10 g/L, yeast powder 5 g/L, and NaCl 10 g/L, which were dissolved in deionized water to a constant volume, sterilized at 121°C for 20 minutes for later use. The solid medium was LB medium with 2% agar added.

After the above-mentioned engineered strain for DAAO enzyme was streaked on a plate and activated, a single colony was picked and inoculated into 5 mL LB liquid medium containing 50 µg/mL kanamycin, and cultured with shaking at 37°C for 12 h. The obtained mixture was transferred at 2% inoculation volume into 150 mL of fresh LB liquid culture medium containing 50 µg/mL kanamycin the same, shaked at 37°C until OD₆₀₀ reached about 0.8, and cooled to 30°C. IPTG was added until the final concentration was 0.5 mM, induction culture was performed for 16 h. After the culture was completed, the culture liquid was centrifuged at 10000 rpm for 10 min, the supernatant was discarded, the bacterial cells were collected, and stored in a -20°C refrigerator for later use.

The bacterial cells collected after the culture was completed were washed twice with 50 mM pH 8.0 phosphate buffer, then resuspended in 50 mL of pH 8.0 phosphate buffer, homogenized and lysed, and the lysate was centrifuged to remove the precipitate to obtain a crude enzyme solution containing the recombinant DAAO enzyme.

### Example 2: Construction of D-amino acid oxidase (DAAO) mutant

### 1. Activation of engineered bacteria and extraction of plasmids

The activated engineered bacteria for DAAO enzyme described in Example 1 was inoculated into a test tube containing 5 mL of LB culture medium, and cultured at 37°C and 200 rpm for 8-12 h. After obtaining the cultured bacterial cells, plasmid extraction was performed according to the operating instructions of the Sangon plasmid extraction kit. The resulted plasmid can be directly used for point mutation, or stored in a -80°C refrigerator for long-term storage.

### 2. Construction of mutant library (position K29, position V42, position E195, position C234, and position V326)

Whole plasmid PCR method was used for gene mutation to obtain mutant genes.

The plasmid extracted in the above step was used as a template, and PCR primer sequences were designed for construction of a mutant library for mutations at positions K29, V42, E195, C234 and V326 of the mutant D-amino acid oxidase sequence to obtain the gene of target mutants. The primer sequences are shown in Table 3:

**Table 3**

| No. | Mutation site and primer name | Primer sequence | SEQ ID NO: |
|---|---|---|---|
| 1 | 29-forward primer | ctttagcccagaagggctatNNKgtgcatgtggttgcccgc | 2 |
| 2 | 29-reverse primer | gcgggcaaccacatgcacMNNatagcccttctgggctaaag | 3 |
| 3 | 42-forward primer | gatctgccggaagacacaNNKgcccagacctttgcaagc | 4 |
| 4 | 42-reverse primer | gcttgcaaaggtctgggcMNNtgtgtcttccggcagatc | 5 |
| 5 | 195-forward primer | gtggaagatcaagaagtgNNKcctatccgcggccaaacag | 6 |
| 6 | 195-reverse primer | ctgtttggccgcggataggMNNcacttcttgatcttccac | 7 |
| 7 | 234-forward primer | ccgggtggtgaagtgatcNNKggtggtacctatttagtg | 8 |
| 8 | 234-reverse primer | cactaaataggtaccaccMNNgatcacttcaccacccgg | 9 |
| 9 | 326-forward primer | ggtaaagcaagccgtaccNNKccggttgttcatgcctatg | 10 |
| 10 | 326-reverse primer | cataggcatgaacaaccggMNNggtacggcttgctttacc | 11 |

Among them, N represents any nucleotide of A, G, C, or T; M represents A or C, and K represents G or T; the desired nucleotide was selected according to the coding nucleotide of the amino acid to which the site was to be mutated. For example, NNK in the K29-forward primer could represent AAG (lysine), AAT (aspartic acid), AGG (arginine acid) or AGT (serine), etc.

The PCR amplification system is:

**Table 4**

| Reagent | Amount of usage (µL) |
|---|---|
| 2× PCR buffer (containing high-fidelity enzyme) | 25 |
| Forward primer (10 µM) | 1 |
| Reverse primer (10 µM) | 1 |
| Template | 1 |
| Deionized water | 22 |

The PCR amplification procedure is as follows:

**Table 5**

| Reaction temperature | Reaction time | Number of cycles |
|---|---|---|
| 95°C | 5min | 1 |
| 95°C | 1min | 30 cycles |
| 50°C | 30s | |
| 72°C | 2min | |
| 72°C | 5min | - |
| 12°C | ∞ | - |

DpnI enzyme was added to digest the PCR product at 37°C for 2 h. After the reaction was completed, the digested PCR product was transformed into *E.coli* BL21 competent cells, which were spread on LB medium containing 50 µg/mL kanamycin, and cultured overnight at 37°C. The bacterial cells were then collected, and the transformants containing the mutant libraries were obtained.

### 3. Combinatorial mutations

The screened beneficial mutations were used to achieve multiple different mutation combinations through overlap PCR to form new mutant transformants.

### Example 3: Preliminary screening of high-throughput mutant library

The transformants obtained in Example 2 were inoculated into a 96-well plate and cultured. IPTG was added to a final concentration of 0.5 mM and induction was performed overnight at 30°C. Afterwards, the bacterial cells were collected, lysed by adding bugbuster protein extraction reagent, centrifuged and the supernatant was taken to obtain the DAAO mutant enzyme solution.

Thermal stability screening method:
the above supernatant containing enzymes was placed in a water bath kettle of 60°C and subj ected to heat treatment for 20 minutes. Then "enzyme activity detection and analysis" was used to screen positive clones.

Enzyme activity detection and analysis: 100 µL of 100 mM substrate (D,L-glufosinate, purchased from Shanghai Aladdin Biochemical Technology Co., Ltd.) with a pH of 8.0 was taken, and 50 µL of chromogenic solution (containing TBHBA (3-hydroxy-2,4,6-tribromobenzoic acid) at 60 mg/mL and 4-AAP (4-aminoantipyrine) at 100 mg/mL) and 25 µL of HRP (horseradish peroxidase, 0.1 mg/mL) were added, finally 25 µL of the above DAAO mutant enzyme solution was added to obtain a 200 µL reaction system for the enzyme plate, and the reaction system was analyzed under the conditions of 30°C and pH 8.0. The absorbance at 510 nm was recorded at 0 min and 20 min, respectively, and the difference was taken. The wild type was used as the reference system to screen positive clones. Positive clones with enzyme activity equivalent to or higher than Enz.01 and enhanced thermal stability were screened out and are shown in Table 6.

**Table 6**

| Enzyme number | Mutation site | Codon |
|---|---|---|
| Enz.1 | / | / |
| Enz.2 | K29G | GGC |
| Enz.3 | K29H | CAC |
| Enz.4 | K29I | ATC |
| Enz.5 | K29N | AAC |
| Enz.6 | K29Q | CAG |
| Enz.7 | K29W | TGG |
| Enz.8 | K29Y | TAC |
| Enz.9 | V42C | TGT |
| Enz.10 | V42D | GAT |
| Enz.11 | V42E | GAA |
| Enz.12 | V42H | CAC |
| Enz.13 | V42P | CCG |
| Enz.14 | V42Y | TAC |
| Enz.15 | E195H | CAC |
| Enz.16 | E195N | AAT |
| Enz.17 | E195Q | CAA |
| Enz.18 | E195Y | TAT |
| Enz.19 | C234L | CTC |
| Enz.20 | V326W | TCG |
| Enz.21 | K29C-V42Y-C234L | 29TGT-42TAC-234CTC |
| Enz.22 | K29G-V42Y-C234L | 29GGC-42TAC-234CTC |
| Enz.23 | K29L-V42Y-C234L | 29TTA-42TAC-234CTC |
| Enz.24 | K29Q-V42Y-E195Y | 29CAG-42TAC-195TAT |
| Enz.25 | K29W-V42Y-E195Y | 29TGG-42TAC-195TAT |
| Enz.26 | K29Y-V42Y-E195Y | 29TAC-42TAC-195TAT |
| Enz.27 | V42Y-C234L | 42TAC-234CTC |
| Enz.28 | V42Y-C234L-V326W | 42TAC-234CTC-326TCG |
| Enz.29 | V42Y-E195Y | 42TAC-195TAT |
| Enz.30 | V42Y-E195Y-C234L | 42TAC-195TAT-234CTC |
| Enz.31 | E195Y-C234L | 195TAT-234CTC |
| Enz.32 | E195Y-C234L-V326W | 195TAT-234CTC-326TCG |
| Enz.33 | E195Y-V326W | 195TAT-326TCG |
| Enz.34 | C234L-V326W | 234CTC-326TCG |

### Example 4: Rescreening of mutants with improved thermal stability

Enzyme activity detection method for rescreening of mutants:
1 mL of 500 mM D,L-glufosinate (ammonium salt), 0.25 mL of DAAO mutant crude enzyme solution after heat treatment (60°C, 20 min), and 1.25 mL of horseradish peroxidase (HRP), 2.5 mL of chromogenic dye solution (containing TBHBA at 60 mg/mL and 4-AAP at 100 mg/mL) were added to form a 5 mL reaction system. The reaction medium was a disodium hydrogen phosphate-sodium dihydrogen phosphate buffer with a pH of 8.0; the mixture was reacted under shaking on a shaking table at 30°C and the reaction liquid was taken and scanned to obtain the absorbance value at 510 nm every 2 minutes; the enzyme reactive dynamic curve of absorbance vs time (min) was plotted, and the enzyme activity was calculated according to the curve slope.

Definition of unit enzyme activity: the amount of enzyme required to generate 1 µmol H₂O₂ per minute under specific reaction conditions (30°C), the unit of enzyme activity is U.

According to the above enzyme activity measurement method, the enzyme activity before and after heat treatment was detected, and the fold improvement of thermal stability relative to Enz.1 was calculated. The results are shown in Table 7 below. * indicates that the thermal stability is improved by 1 to 1.2 folds (excluding 1.2), ** indicates that the thermal stability is improved by 1.2 to 2 folds (excluding 2), *** indicates that the thermal stability is improved by 2 folds or more. Method for calculating the fold improvement of thermal stability: ratio of enzyme activity after heat treatment to enzyme activity before treatment of the mutant/ratio of enzyme activity after heat treatment to enzyme activity before treatment of Enz.01.

**Table 7**

| Enzyme number | Mutant (site) | Fold improvement of thermal stability |
|---|---|---|
| Enz.01 | / | / |
| Enz.02 | K29G | * |
| Enz.03 | K29H | *** |
| Enz.04 | K29I | *** |
| Enz.05 | K29N | ** |
| Enz.06 | K29Q | *** |
| Enz.07 | K29W | ** |
| Enz.08 | K29Y | ** |
| Enz.09 | V42C | ** |
| Enz.10 | V42D | ** |
| Enz.11 | V42E | *** |
| Enz.12 | V42H | *** |
| Enz.13 | V42P | * |
| Enz.14 | V42Y | *** |
| Enz.15 | E195H | * |
| Enz.16 | E195N | ** |
| Enz.17 | E195Q | ** |
| Enz.18 | E195Y | * |
| Enz.19 | C234L | ** |
| Enz.20 | V326W | ** |
| Enz.21 | K29C-V42Y-C234L | *** |
| Enz.22 | K29G-V42Y-C234L | *** |
| Enz.23 | K29L-V42Y-C234L | *** |
| Enz.24 | K29Q-V42Y-E195Y | *** |
| Enz.25 | K29W-V42Y-E195Y | *** |
| Enz.26 | K29Y-V42Y-E195Y | *** |
| Enz.27 | V42Y-C234L | *** |
| Enz.28 | V42Y-C234L-V326W | *** |
| Enz.29 | V42Y-E195Y | *** |
| Enz.30 | V42Y-E195Y-C234L | *** |
| Enz.31 | E195Y-C234L | *** |
| Enz.32 | E195Y-C234L-V326W | *** |
| Enz.33 | E195Y-V326W | ** |
| Enz.34 | C234L-V326W | *** |

As shown in the table above, it shows that most single-point mutant enzymes and combined mutant enzymes have significant effects on improving the thermal stability, and Enz.27, Enz.29, and Enz.32 have better effects.

### Example 5: Preparation of L-glufosinate

The reaction route involved in the present disclosure is as follows:

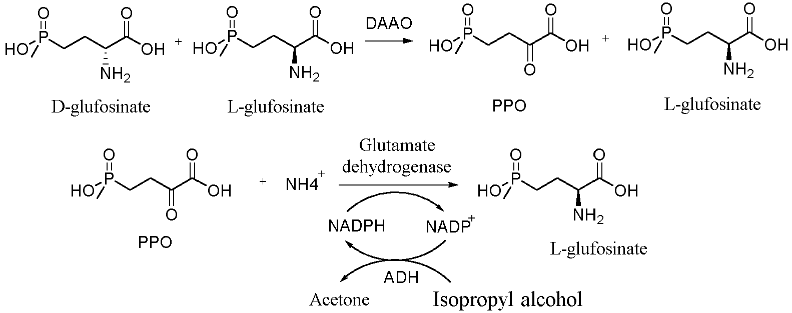

Based on the Cyclopentanol dehydrogenase gene sequence derived from *Lactobacillus brevis* (*Lactobacillus brevis* KB290) (GenBank accession number: BAN05992.1), the alcohol dehydrogenase gene was fully synthesized.

LB liquid culture medium composition: peptone 10 g/L, yeast powder 5 g/L, and NaCl 10 g/L, which were dissolved in deionized water to a constant volume, sterilized at 121°C for 20 minutes for later use.

The alcohol dehydrogenase gene was linked to pET28a, and the sites were digested with NdeI & HindIII. The recombinant vector was transformed into the host *E. coli* BL21 (DE3) competent cells to obtain an engineered strain containing the alcohol dehydrogenase gene. After the engineered strain containing the alcohol dehydrogenase gene was streaked on a plate and activated, a single colony was picked and inoculated into 5 mL LB liquid medium containing 50 µg/mL kanamycin, and cultured with shaking at 37°C for 12 h. The obtained mixture was transferred at 2% inoculation volume into 50 ml of fresh LB liquid culture medium containing 50 µg/mL kanamycin the same, shaken at 37°C until OD₆₀₀ reached about 0.8. IPTG was added until the final concentration was 0.5 mM, induction culture was performed at 18°C for 16 h. After the culture was completed, the culture liquid was centrifuged at 10000 rpm for 10 min, the supernatant was discarded, the bacterial cells were collected, and stored in a -20°C ultra-low temperature refrigerator for later use.

Preparation of enzyme solution: The bacterial cells with high enzyme activity (enzyme numbers: Enz.27, Enz.29, and Enz.32) selected in Example 4 were placed in a 50 mmol/L ammonium phosphate buffer at pH 7.0 and homogenized. The ratio of bacterial cells to buffer was 1 : 5 (g : mL), and after homogenization, flocculant was added to flocculate the supernatant.

4600 g of water was added to a 10 L jacketed reaction tank, 0.28 g of ammonium dihydrogen phosphate and 5.57 g of diammonium hydrogen phosphate were added, stirred and dissolved, and then 400 g of D,L-glufosinate (ammonium salt) was added into the reaction tank, stirred and dissolved. The pH of the solution was adjusted to 7.9-8.1 with ammonia water, 5 g of catalase (enzyme activity 800,000 µ/g) was added into the reaction tank, and 1000 mL (200 g of bacterial cells) of mutant DAAO enzyme solution (Enz.27, Enz.29 or Enz.32) was added. Air-blowing was performed, and the ventilation volume was controlled at 1 reaction volume per minute. The temperature was controlled at 25°C and the reaction was carried out for 20 h.

The temperature was controlled to 30-33°C, 0.4 g of NADP⁺, 78 g of isopropyl alcohol and 50 g of ammonium chloride were added, the pH was adjusted to 8.4-8.6 with ammonia water, and 0.35 g of bacterial cells containing alcohol dehydrogenase (ADH) were added. When the temperature and pH were normal, 160 g of bacterial cells containing glutamate dehydrogenase (i.e., Mutants 1-4 in CN 201910434350.1 of our company) was added into the tank to start the reaction. The reaction was carried out for 6 h and the PPO residual amount was detected to be ≤ 2%.

**Table 8**

| Enzyme number | Unit enzyme activity U/mL | Ion pair-PPO% 2 h | ee value 24 h |
|---|---|---|---|
| Enz.1 | 20.25 | 42.55% | 95.34% |
| Enz.27 | 22.05 | 42.09% | 99.14% |
| Enz.29 | 19.37 | 43.27% | 98.44% |
| Enz.32 | 21.03 | 43.75% | 98.18% |

The reaction results are shown in Table 8; The ee values of Enz.27, Enz.29 and Enz.32 after 24 h of reaction are all no less than 98%, which is significantly better than Enz. 1.

## Claims

1. A D-amino acid oxidase, wherein the amino acid sequence of the D-amino acid oxidase has one or more amino acid residue difference selected from the following as compared to SEQ ID NO: 1:
K29G/H/I/N/Q/W/Y/C/L; V42C/D/E/H/Y; E195N/Y/Q; C234L andV326W;
and the D-amino acid oxidase has an activity and/or a thermal stability not lower than that of a D-amino acid oxidase of the amino acid sequence as set forth in SEQ ID NO: 1.

2. The D-amino acid oxidase according to claim 1, wherein, the amino acid sequence of the D-amino acid oxidase comprises two or more amino acid residue differences selected from the following as compared to SEQ ID NO: 1: V42Y, E195Y, V326W, C234L.

3. The D-amino acid oxidase according to claim 2, wherein, the D-amino acid oxidase comprises the amino acid residue difference of C234L as compared to SEQ ID NO: 1, and further comprises one or two amino acid residue differences of V42Y, E195Y and V326W;
preferably, the D-amino acid oxidase comprises the amino acid residue difference selected from any of the following groups as compared to SEQ ID NO: 1:
C234L and V42Y;
C234L and E195Y;
C234L and V326W;
C234L, K29C and V42Y;
C234L, K29G and V42Y;
C234L, K29L and V42Y;
C234L, V326W and V42Y;
C234L, V42Y and E195Y;
and C234L, E195Y and V326W.

4. The D-amino acid oxidase according to claim 2, wherein, the D-amino acid oxidase comprises the amino acid difference of E195Y as compared to SEQ ID NO: 1, and further comprises the amino acid residue difference of V42Y or V326W;
preferably, the D-amino acid oxidase comprises the amino acid residue difference selected from any of the following groups as compared to SEQ ID NO: 1:
E195Y and V42Y;
E195Y and V326W;
E195Y, K29Q and V42Y;
E195Y, K29W and V42Y;
and E195Y, K29Y and V42Y

5. The D-amino acid oxidase according to claim 1, wherein, the amino acid sequence of the D-amino acid oxidase comprises the amino acid residue difference selected from any of the following groups as compared to SEQ ID NO: 1:
K29G; K29H; K29I; K29N; K29Q; K29W; K29Y; V42C; V42D; V42E; V42H; V42P; V42Y; E195H; E195N; E195Y; E195Q; C234L; and V326W.

6. An isolated nucleic acid encoding the D-amino acid oxidase according to any one of claims 1-5.

7. A recombinant expression vector comprising the nucleic acid according to claim 6.

8. A transformant comprising the nucleic acid according to claim 6 or the recombinant expression vector according to claim 7; preferably, the host cell of the transformant is *Escherichia coli,* such as *E.coli* BL21 (DE3).

9. A method for preparing the D-amino acid oxidase according to any one of claims 1-5, comprising culturing the transformant according to claim 8 under a condition suitable for expression of the D-amino acid oxidase.

10. A method for preparing 2-oxo-4-(hydroxymethylphosphinyl)butyric acid or a salt thereof, comprising the step of subjecting a substrate to an oxidation reaction in the presence of the D-amino acid oxidase according to any one of claims 1-5 to obtain the 2-oxo-4-(hydroxymethylphosphinyl)butyric acid or the salt thereof,
preferably,
the substrate is D-glufosinate or a salt thereof, the D-glufosinate or the salt thereof may exist alone or together with L-glufosinate or a salt thereof; for example, the substrate exists in the form of racemic glufosinate or a salt thereof;
and/or, the oxidation reaction is carried out under a ventilation condition; the ventilation is preferably performed with air or oxygen; the ventilation rate is preferably 0.5VVM-1.5VVM, such as 1VVM;
and/or, the oxidation reaction is carried out in the presence of catalase;
and/or, the D-amino acid oxidase exists in the form of bacterial cells containing the D-amino acid oxidase, a crude enzyme, a pure enzyme or an immobilized enzyme of the D-amino acid oxidase;
and/or, the concentration of the substrate is 0.1-0.5 mol/L; preferably, 0.17 mol/L;
and/or, the pH of the reaction system of the oxidation reaction is 7-9, preferably 8;
and/or, the temperature of the reaction system of the oxidation reaction is 20-50°C, preferably 25°C;
more preferably, the mass ratio of the bacterial cells containing the D-amino acid oxidase to the substrate is 1 : (0.5-3), for example, 1 : 1; and/or, the mass ratio of the catalase to the bacterial cells containing the D-amino acid oxidase is 1 : (20-60); for example, 1 : 40.

11. The method according to claim 10, wherein, the reaction system of the oxidation reaction comprises a buffer, and the buffer is preferably a phosphate buffer, such as ammonium dihydrogen phosphate and diammonium hydrogen phosphate, disodium hydrogen phosphate and sodium dihydrogen phosphate.

12. A method for preparing L-glufosinate or a salt thereof, comprising the following steps:
(1) obtaining 2-oxo-4-(hydroxymethylphosphinyl)butyric acid or a salt thereof by adopting the method according to claim 10 or 11;
(2) in the presence of glutamate dehydrogenase, inorganic amino donor and reduced coenzyme, performing an ammoniation reaction with the 2-oxo-4-(hydroxymethylphosphinyl)butyric acid or the salt thereof obtained in step (1) to obtain the L-glufosinate or the salt thereof.

13. The method according to claim 12, wherein,
the reduced coenzyme is NADPH or NADH;
and/or, the inorganic amino donor is one or more of ammonia, ammonium sulfate, ammonium chloride, diammonium hydrogen phosphate, ammonium acetate, ammonium formate and ammonium bicarbonate, and the form of the ammonia when used is preferably ammonia water;
and/or, the pH of the reaction system of the ammoniation reaction is 7-10, preferably 8.4-8.6;
and/or, the reaction temperature of the ammoniation reaction is 28-35°C, preferably 30-33°C.

14. Use of the D-amino acid oxidase according to any one of claims 1-5 in the preparation of L-glufosinate or a salt thereof, and 2-oxo-4-(hydroxymethylphosphinyl)butyric acid or a salt thereof.
